(19) Europäisches Patentamt
European Patent Office
Office européen des brevets

(11) Numéro de publication: **0 446 094 B1**

(12) **FASCICULE DE BREVET EUROPEEN**

(45) Date de publication de fascicule du brevet: **22.03.95**

(51) Int. Cl.[6]: **C08L 91/06**, C09G 1/08, A61K 7/06

(21) Numéro de dépôt: **91400486.6**

(22) Date de dépôt: **22.02.91**

(54) **Compositions fluides contenant une microdispersion de cire et d'un tensio-actif cationique, leur preparation et leur utilisation.**

(30) Priorité: **23.02.90 FR 9002295**

(43) Date de publication de la demande:
**11.09.91 Bulletin 91/37**

(45) Mention de la délivrance du brevet:
**22.03.95 Bulletin 95/12**

(84) Etats contractants désignés:
**AT BE CH DE DK ES FR GB GR IT LI NL SE**

(56) Documents cités:
**FR-A- 2 369 340**
**GB-A- 1 312 654**

**PATENT ABSTRACTS OF JAPAN, vol. 13, no. 313 (C-618)[3661], 17 juillet 1989;& JP-A-1 096 247**

(73) Titulaire: **L'OREAL**
**14, Rue Royale**
**F-75008 Paris (FR)**

(72) Inventeur: **Vanlerberghe, Guy**
**Rue du Général de Gaulle**
**F-77410 Montjay la Tour (FR)**
Inventeur: **Nicolas-Morgantini Luc**
**5, rue du Vignet**
**F-60810 Rully (FR)**
Inventeur: **Lety, Alain**
**9, rue de Metz**
**F-77400 Lagny S/Marne (FR)**

(74) Mandataire: **Tonnellier, Jean-Claude et al**
**Cabinet Nony & Cie.**
**29, rue Cambacérès**
**F-75008 Paris (FR)**

**EP 0 446 094 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des compositions fluides contenant une microdispersion de cire, ainsi qu'un procédé de préparation de ces compositions, et leur utilisation.

La demande de brevet français n°77.32762 (n° de publication 2.369.340) décrit des compositions parfumées solides, liquides ou pâteuses, contenant des particules fines d'un diamètre moyen de 0,1 à 200 µm comprenant jusqu'à 95% d'une cire ayant un point de fusion de 38 à 150°C. Ces particules sont destinées à servir de véhicule pour un parfum, le parfum représentant de 1 à 75% du poids des particules. La taille des particules est de préférence comprise entre 1 et 100µm, et plus spécialement entre 5 et 50µm. Ces particules sont incorporées dans un support ou une composition contenant un tensio-actif cationique. On procède par fragmentation mécanique des constituants des particules solides puis broyage, éventuellement en présence des autres constituants dans le cas où la composition à préparer est une composition liquide. Le produit broyé peut également être incorporé par mélange à un support solide ou liquide. Les compositions obtenues, qui se présentent sous la forme de pâtes, de crèmes ou de compositions liquides, sont utilisables pour le conditionnement des textiles, ainsi que pour le conditionnement de la chevelure.

On sait par ailleurs qu'il est possible d'obtenir avec certaines huiles des microémulsions et avec certaines cires des microdispersions, stables et, pour ces dernières, diluables à l'eau indéfiniment, sans agrégation ni sédimentation des particules en suspension. Les microdispersions de cire sont obtenues par fusion de la cire en présence d'un tensio-actif anionique ou non-ionique, et éventuellement d'une partie de l'eau, puis addition progressive d'eau chaude avec agitation. On observe la formation intermédiaire d'une émulsion du type eau-dans-l'huile, suivie d'une inversion de phase avec obtention finale d'une émulsion du type huile-dans-l'eau. Au refroidissement, on obtient une microdispersion stable de particules colloïdales solides de cire ; voir par exemple "Microemulsions Theory and Practice", L.M. Prince Ed., Academic Press (1977) pages 21-32. Ces microdispersions de cire sont utilisées notamment pour faire briller les articles en cuir et les revêtements de sol en matière plastique.

Les cires sont des substances naturelles (animales ou végétales) ou synthétiques, solides à la température ordinaire (21°C), ayant généralement une certaine plasticité, qui sont insolubles dans l'eau, solubles dans les huiles, et qui sont capables de former des films hydrofuges. Sur la définition des cires, on peut citer par exemple P.D. Dorgan, Drug and Cosmetic Industry, Decembre 1983, pages 30-33, et Handbook of Cosmetic Science, H.W. Hibbot ed., Pergamon Press, Oxford (1963) p60.

Dans la présente demande, on appelle "microdispersion" une dispersion qui est obtenue par le procédé qui vient d'être rappelé, ou par un procédé analogue.

On a maintenant découvert que de telles microdispersions de cire peuvent être obtenues en remplaçant, dans le procédé qui vient d'être décrit, l'agent tensioactif anionique ou non-ionique par un agent tensio-actif cationique. Les nouvelles compositions obtenues présentent des propriétés intéressantes. Elles peuvent être utilisées par exemple comme agents d'entretien (produits auto-lustrants) des articles en cuir, des revêtements de sol (notamment en plastique), ou des meubles, auxquels elles confèrent un brillant durable, ou encore comme agents de conditionnement des textiles.

Ces compositions peuvent aussi être utilisées comme supports de compositions cosmétiques ou comme compositions cosmétiques, et en particulier comme lotions pour cheveux. Ces lotions présentent notamment la propriété surprenante de ne pas conférer aux cheveux un aspect gras mais, au contraire, de retarder l'apparition de l'aspect gras des cheveux, bien qu'elles contiennent comme ingrédient actif principal une cire, c'est-à-dire une substance classée parmi les matières grasses.

En outre, il n'était pas évident qu'il serait possible d'obtenir des microémulsions de cire en présence d'un émulsionnant cationique. On sait en effet que les cires qui sont impérativement présentes dans la composition et qui sont utilisées seules ou en mélange avec d'autres cires, contiennent pratiquement toujours des acides gras libres. On pouvait donc s'attendre à une incompatibilité des tensioactifs cationiques avec ces cires ayant un caractère anionique.

La présente invention a donc pour objet une composition fluide se présentant sous la forme d'une dispersion de cire dans un véhicule liquide constitué d'une seule phase, dans laquelle la phase dispersée est une microdispersion stable constituée de particules de dimensions inférieures à O,5µm, lesdites particules sont constituées essentiellement d'un mélange d'au moins une cire, d'au moins un agent émulsionnant et éventuellement d'au moins une huile et/ou d'au moins un ingrédient actif liposoluble, ledit mélange ayant un point de fusion finissante supérieur à 50°C et inférieur à 100°C, ladite composition contenant, en poids, de 0,1 à 40 % de cire et de 0,01 à 25 % dudit agent émulsionnant, ledit agent émulsionnant est un émulsionnant cationique, et la concentration de ladite huile, lorsqu'elle est présente, est inférieure ou égale à 30 % en poids par rapport au poids de la cire ou du mélange de cires.

La cire ou le mélange de cires utilisé selon l'invention doit donc être capable de donner, en association avec des agents émulsionnants cationiques, selon le procédé décrit ci-dessus, des microdispersions stables ayant des dimensions de particules inférieures à 0,5 micromètre. Les cires ou mélanges de cires utilisables peuvent être choisis par de simples expériences de routine.

Dans des modes de réalisation particuliers, les compositions de l'invention peuvent encore présenter les caractéristiques suivantes, prises isolément ou en combinaison :

- la cire est une cire choisie parmi la cire de Carnauba, la cire de Candelilla, la cire d'Alfa et leurs mélanges ;
- la cire contient, outre de la cire de Carnauba ou de la cire de Candelilla ou leurs mélanges, une autre cire ou un mélange d'autres cires, par exemple une cire de paraffine, l'ozokérite, la cire de jojoba hydrogénée, la cire d'abeille éventuellement estérifiée, la cire de riz ou des céramides, y compris des céramides insaturés ; la proportion pondérale de cire de Carnauba et/ou de Candelilla, dans de tels mélanges, est de préférence supérieure ou égale à 50 % ;
- la proportion de cire dans la composition peut varier de 0,1 à 20 % en poids, et en particulier de 1 à 20 % ;
- ledit agent émulsionnant cationique est caractérisé par une valeur de HLB comprise entre 11 et 16 ; les tensioactifs cationiques préférés sont des dérivés d'ammonium quaternaire ; des exemples de tels tensioactifs cationiques sont donnés dans la partie expérimentale ci-après.
- l'agent émulsionnant est présent à une concentration de 0,1 à 10 % en poids ;
- ledit véhicule liquide contient de 70 % à 100 % en poids d'eau, par rapport au poids de la phase liquide ;
- le véhicule aqueux est constitué par de l'eau ;
- le rapport pondéral cire/émulsionnant peut varier dans la gamme de 1 à 20, notamment de 2 à 10 ;
- les ingrédients actifs liposolubles sont par exemple des colorants liposolubles, ou des filtres solaires (substances capables de conférer une protection à un substrat tel que la peau et/ou les cheveux contre les effets nocifs du rayonnement ultraviolet) liposolubles ; la concentration du ou des ingrédients actifs liposolubles, lorsqu'ils sont présents, peut aller jusqu'à 30 % (généralement jusquà 10 %) en poids par rapport à la cire ou au mélange de cires ;
- au moins un composé amphiphile (non émulsionnant des cires) tel que le cholestérol, les alcools gras contenant au moins 12 carbones etc., peut être associé à la cire ; la concentration en composés amphiphiles peut atteindre jusqu'à 30 % (en particulier jusqu'à 10 %) en poids par rapport à la cire ;
- la composition est exempte d'huile, ou bien la concentration de l'huile, lorsqu'elle est présente est inférieure ou égale à 10 % en poids par rapport au poids de la cire ou du mélange de cires ; parmi les huiles utilisables, on citera notamment celles qui sont mentionnées dans la partie expérimentale ci-dessous ;
- la proportion pondérale totale de cire et de composés amphiphiles non émulsionnants éventuellement présents, dans les particules (agents émulsionnants mis à part), est généralement supérieure à 90 %, et le plus souvent supérieure à 95 %, le reste étant constitué par les huiles et les ingrédients liposolubles éventuellement présents.

De préférence, les compositions de l'invention ne contiennent pas de tensio-actifs non ioniques et/ou anioniques.

Les cires végétales de Carnauba (extraites de Copernica Cerifera), de Candelilla (extraites de Euphorbies Cerifera et de Pedilantus Pavonis), et d'Alfa (extraites de Stipa Tenacissima) sont des produits commerciaux.

Les céramides sont les principaux lipides constitutifs des espaces intercornéocytaires du stratum corneum. Ils ont été décrits, en particulier par Downing dans Science 1982 p 1261-2 vol.18. Les céramides sont utilisés notamment dans les compositions cosmétiques comme agents antivieillissement et comme agents hydratants ; voir par exemple la demande de brevet japonais 87.176907. Dans les compositions capillaires, ils agissent comme agents de protection du cheveu ; voir par exemple la demande de brevet européen 0278505.

Ces céramides sont difficiles à disperser dans les compositions cosmétiques. Grâce à la présente invention, il est possible de les disperser à des concentrations élevées.

Les colorants liposolubles éventuellement présents dans la composition sont par exemple :

- Nitro 1-amino-3 isopropyl 4 aniline,
- Nitro 1-méthyl-2 méthylamino 3 méthyl 4 aniline,
- Nitro 3 butylamino 4 phénol,
- Hydroxy-4 méthyl-3 phénylazo benzène,

- Le produit de formule :

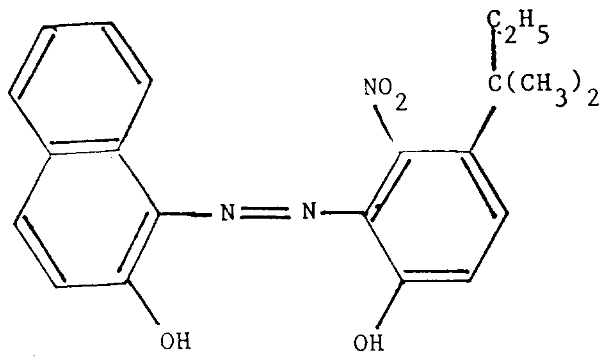

Parmi les filtres solaires liposolubles éventuellement présents dans la composition de l'invention, on peut citer notamment les suivants, qui sont disponibles dans le commerce :

- le 3-benzylidène-d,l-camphre,
- le 3-(4'-méthylbenzylidène)-d, l-camphre,
- le 4-(diméthylamino)-benzoate d'amyle,
- le p-méthoxycinnamate d'amyle et d'isoamyle,
- le salicylate de méthyle,

L'un des avantages de la composition de l'invention est de permettre l'utilisation de ces ingrédients liposolubles en milieu aqueux.

Les compositions de l'invention peuvent également constituer des supports de compositions, auxquels on ajoute d'autres ingrédients.

Les compositions obtenues selon l'invention peuvent contenir, notamment, un ou plusieurs ingrédients secondaires usuels tels que des agents épaississants, des agents modificateurs de pH, des parfums, des agents conservateurs, ou des agents antistatiques.

Les agents épaississants doivent être compatibles avec les tensioactifs cationiques utilisés. Ils sont utilisés à une concentration telle que la viscosité de la composition soit au plus égale à 25 poises (soit 2,5 Pa.s) environ à 25 °C (viscosimètre Contraves, corps de mesure n° 3 ; temps de rotation 10 minutes, à 200 tours/min).

Les parfums utilisables sont des parfums usuels, solubles dans la cire ou dispersibles ou solubles dans l'eau, en particulier ceux qui sont dispersibles ou solubles dans l'eau. Ils sont généralement utilisés à une concentration ne dépassant pas 5 % en poids.

Les agents conservateurs éventuellement présents sont par exemple la diméthyloldiméthylhydantoïne et les dérivés d'imidazolidinyl urée. Ils sont utilisés aux concentrations efficaces usuelles.

Le pH des compositions selon l'invention peut varier par exemple de 1 à 13 et de préférences de 3 à 11. Le pH peut éventuellement être ajusté a l'aide d'un agent modificateur de pH usuel.

Les compositions selon l'invention sont obtenues par formation à chaud d'une microémulsion. Plus précisément, ces compositions sont obtenues par un procédé principalement caractérisé par le fait que l'on chauffe le mélange de cire et d'émulsionnant, additionné éventuellement d'huile et/ou des substances liposolubles, à une température supérieure à la température de fusion du mélange et non supérieure à 100 °C, éventuellement en présence d'une partie de l'eau, jusqu'à fusion complète, que l'on ajoute progressivement l'eau, ou le restant de l'eau, portée à une température au moins égale à ladite température, en agitant, jusqu'à formation d'une microémulsion de cire dans une phase continue aqueuse, qui par refroidissement jusqu'à la température ambiante conduit à une microdispersion de cire.

Les ingrédients secondaires éventuellement présents dans la composition sont ajoutés selon les cas soit dans les produits de départ, soit dans la composition terminée. Les ingrédients liposolubles sont généralement ajoutés à la cire, avant la réalisation de la microdispersion.

Les ingrédients hydrosolubles non volatils peuvent être ajoutés éventuellement dans l'eau utilisée pour réaliser la microdispersion.

Les compositions auto-lustrantes pour articles en cuir, meubles ou revêtements de sol, obtenues selon l'invention, sont appliquées selon les méthodes usuelles, à l'aide d'un applicateur absorbant, par exemple en feutre ou en mousse expansée de matière plastique. On peut aussi appliquer la composition par

pulvérisation et étaler en frottant la surface traitée avec un tissu doux.

Les compositions de conditionnement de textiles obtenues selon l'invention sont appliquées par exemple par mélange au liquide de rinçage.

Les compositions cosmétiques selon l'invention peuvent notamment être utilisées comme lotions coiffantes et aussi comme lotions destinées à améliorer l'aspect des cheveux chez les sujets ayant les cheveux gras.

Elles peuvent être appliquées sur cheveux secs et propres, et aussi avant ou après un shampooing. Elles peuvent être rincées ou non rincées.

Lorsqu'elles sont appliquées avant ou après un shampooing, l'application étant suivie ou non d'un rinçage à l'eau, elles disciplinent les cheveux et communiquent à la coiffure de la tenue et du volume. En outre, elles retardent le phénomène de regraissage des cheveux observé chez les sujets ayant les cheveux gras.

Pour éviter ce phénomène de regraissage, on peut appliquer la composition de l'invention sur les cheveux séchés après lavage, et en particulier sur la partie des cheveux voisine de la racine. Dans ce cas, la composition n'est pas rincée. On observe que, malgré l'absence de rinçage, la composition ne communique pas aux cheveux un toucher poisseux, et ne provoque pas de phénomène de collage des cheveux.

Malgré la présence d'une cire dans la composition, aucun aspect gras n'est communiqué aux cheveux, même en l'absence de rinçage. En outre, malgré la présence d'une forte proportion d'eau dans la composition, le séchage ne pose pas de problèmes et s'effectue rapidement.

Quand les compositions contiennent un agent colorant liposoluble, elles peuvent être utilisées comme compositions de teinture pour cheveux.

Il convient encore de noter que les supports de compositions selon l'invention, qui sont diluables en toutes proportions avec de l'eau, peuvent être réalisés sous forme de supports de compositions concentrés contenant des proportions élevées de cire, par exemple de 1 à 40 % en poids.

Ces supports de compositions concentrés peuvent être dilués au moment de l'emploi, pour obtenir une concentration en cire pouvant aller par exemple de 0,1 à 10 %.

On peut également ajouter (après dilution, dans le cas des compositions concentrées) des ingrédients secondaires.

Les supports de compositions obtenus selon l'invention sont donc d'une part les microdispersions concentrées mentionnées ci-dessus, à diluer au moment de l'emploi, et d'autre part les microdispersions non concentrées (contenant au moins l'eau, la cire et le tensio-actif) auxquelles on peut ajouter, au moment de l'emploi, des ingrédients secondaires.

L'invention a également pour objet un procédé de traitement cosmétique des cheveux destiné à améliorer la tenue et le volume de la coiffure et/ou à supprimer ou à retarder l'apparition de l'aspect gras des cheveux, caractérisé par le fait que l'on applique, au moins sur la partie des cheveux voisine de la racine, une composition telle que définie précédemment, en quantité suffisante pour imprégner les cheveux ou les parties de cheveux à traiter.

Ce procédé de traitement cosmétique des cheveux est mis en oeuvre comme cela a été indiqué ci-dessus.

Les exemples suivants illustrent l'invention sans toutefois la limiter.

EXEMPLES 1 A 20 :

Exemples de préparation de microdispersions de cire

La procédure de préparation est la suivante :

On mélange la cire et l'agent émulsionnant cationique et l'on chauffe le mélange à 90°C environ en agitant doucement pour obtenir une bonne homogénéisation. On incorpore ensuite sous agitation l'eau préalablement chauffée à 90°C et on obtient une microémulsion. La microémulsion de cire est ensuite ramenée à température ambiante et forme alors une microdispersion.

Les résultats obtenus avec la cire de Carnauba (10 % en poids, par rapport au poids total de la composition) et divers émulsionnants cationiques sont résumés dans le tableau I.

Les mesures granulométriques concernant les particules en suspension (diamètre hydrodynamique moyen) ont été effectuées par diffusion quasi-élastique de la lumière à l'aide d'un granulomètre laser Coulter N4 à 25°C.

TABLEAU I

| EXEMPLES | EMULSIONNANT | CONCENTRATION PONDERALE (%) | DIAMETRE MOYEN des particules (nm) |
|---|---|---|---|
| 1<br>2<br>3<br>4 | bromure de cétyltriméthyl ammonium (CTA) | 3,33<br>3,79<br>2,84<br>3,00 | 68<br>60<br>149<br>74 |
| 5 | chlorure de CTA | 3,33 | 106 |
| 6 | oxalate de CTA | 3,42 | 79 |
| 7 | gallate de CTA | 4,72 | 50 |
| 8<br>9<br>10 | salicylate de CTA<br>salicylate de CTA<br>salicylate de CTA | 1,75<br>2,63<br>4,38 | 162<br>41<br>15 |
| 11 | bromure de cétyl-pyridinium (CP) | 4,18 | 43 |
| 12 | chlorure de CP | 3,72 | 45 |
| 13 | chlorure de cétyl diméthylbenzyl ammonium (CDBA) | 4,31 | 25 |
| 14 | salicylate de cétyl-azabicyclo-octazonium | 4,94 | 30 |
| 15<br>16<br>17<br>18<br>19<br>20 | Arquad 16-50 *<br>Arquad 18-50 *<br>Arquad T-50 *<br>Arquad 2C-75 *<br>Ethoquad C/12 *<br>Ethoquad 0/12 * | 3,33<br>3,62<br>3,62<br>4,35<br>2,50<br>2,50 | 286<br>303<br>243<br>255<br>350<br>106 |

* Nom commercial de dérivés d'ammonium quaternaire à chaîne grasse commercialisés par Armak Chemicals.

EXEMPLES 21 à 27

De façon analogue, on a préparé des microdispersions à 15 % de cire de Carnauba. Les résultats sont résumés dans le tableau II.

TABLEAU II

| EXEMPLES | EMULSIONNANT | CONCENTRATION PONDERALE (%) | DIAMETRE MOYEN des particules (nm) |
|---|---|---|---|
| 21<br>22<br>23<br>24 | bromure de CTA<br>bromure de CTA<br>bromure de CTA<br>bromure de CTA | 2,27<br>3,41<br>5,00<br>5,68 | 179<br>80<br>55<br>51 |
| 25<br>26 | chlorure de cétyl/ stéaryldiméthyl éthanolammonium | 3,51<br>5,00 | 83<br>71 |
| 27 | bromure de myristyltriméthyl ammonium | 3,41 | 147 |

EXEMPLES 28 à 40

De façon analogue, on a préparé des microdispersions à 10 % de mélanges de cire. Le tensio-actif cationique était dans chaque cas le bromure de CTA (3,79 % en poids). Les résultats sont résumés dans le tableau III.

TABLEAU III

| EXEMPLES | CIRES % en poids | | DIAMETRE MOYEN des particules (nm) |
|---|---|---|---|
| | Carnauba + Paraffine | | |
| 28<br>29<br>30 | 9<br>8<br>7 | 1<br>2<br>3 | 94<br>124<br>160 |
| | Carnauba + Ozokérite * | | |
| 31<br>32 | 9<br>7 | 1<br>3 | 94<br>195 |
| | Carnauba + Candelilla | | |
| 33<br>34 | 9<br>7 | 1<br>3 | 67<br>136 |
| | Carnauba + cire de Jojoba hydrogénée* | | |
| 35 | 9 | 1 | 104 |
| | Carnauba + cire de riz | | |
| 36<br>37<br>38 | 9<br>7<br>5 | 1<br>3<br>5 | 97<br>173<br>197 |
| | Carnauba + Cera Bellina** | | |
| 39<br>40 | 9<br>7 | 1<br>3 | 106<br>138 |

* Commercialisée par Quest International
** Cire d'abeille estérifiée (Quest International)

EXEMPLES 41 à 43

On a préparé des microdispersions de cires ayant la composition pondérale suivante :

| | |
|---|---|
| Carnauba | 10 % |
| Additif lipophile | x % |
| Bromure de CTA | 3,79 % |
| Eau q.s.p. | 100 % |

Les résultats sont résumés dans le tableau suivant :

TABLEAU IV

| EXEMPLES | ADDITIF LIPOPHILE x = | DIAMETRE MOYEN (nm) |
|---|---|---|
| 41 | Colorant* 1% | 29 |
| 42 | Parsol MCX 3% | 350 |
| 43 | Parsol MCX 2,4%<br>Uvinul M40 0,6% | 256 |

* isopropyl-2 nitro-6 aniline

Le Parsol MCX est la dénomination commerciale de l'octyl méthoxy cinnamate, commercialisé par GIVAUDAN, et utilisé comme agent de filtration des ultraviolets ("filtre solaire").

Uvinul M40 est la dénomination commerciale de la benzophénone-3, commercialisée par BASF et utilisée comme filtre solaire.

La procédure de préparation est la suivante : on mélange la cire, l'agent émulsionnant cationique et les produits actifs liposolubles (colorant ou filtre solaire), puis on procède comme à l'exemple 1.

EXEMPLES 44 à 46

De façon analogue à celle décrite aux exemples 41 à 43, on a préparé des microdispersions de cires ayant la composition pondérale suivante :

| | |
|---|---|
| Carnauba | x % |
| Huile | y % |
| Bromure de CTA | 3,79% |
| Eau | 86,21% |
| avec x + y = 10 | |

Les résultats sont résumés dan le tableau suivant :

TABLEAU V

| EXEMPLES | CIRE x = | HUILE y = | DIAMETRE DES PARTICULES DES PARTICULES (nm) |
|---|---|---|---|
| | Carnauba | huile de paraffine | |
| 44a<br>44b | 9<br>7 | 1<br>3 | 43<br>106 |
| | Carnauba | DV | |
| 45a<br>45b | 9<br>7 | 1<br>3 | 29<br>39 |
| | Carnauba | huile de tournesol | |
| 46a<br>46b | 9<br>7 | 1<br>3 | 85<br>174 |
| DV : (Ethyl-2 hexyloxy)-3 hexadecanoyloxy-1 propanol-2, décrit dans le brevet FR 2222351. | | | |

EXEMPLES 47 à 49

De façon analogue, on a préparé des microdispersions de cire ayant la composition pondérale suivante :

| | |
|---|---|
| Carnauba | x % |
| Céramide et/ou cholestérol | y % |
| Bromure de CTA | 3,79 % |
| Eau | 86,21 % |
| avec x + y = 10 | |

Les résultats sont résumés dans le tableau suivant :

TABLEAU VI

| EXEMPLES | CIRE + COMPOSE LIPOPHILE % en poids | DIAMETRE MOYEN des particules (nm) |
|---|---|---|
| | Carnauba + DVA* | |
| 47 | 8 2 | 123 |
| | Carnauba + cholestérol | |
| 48a | 9 1 | 91 |
| 48b | 8 2 | 196 |
| | Carnauba + DVA + cholestérol | |
| 49 | 8 1 1 | 129 |

*DVA : Céramide de formule $C_{15}H_{31}CHOHCH(CH_2OH)NHCOC_{15}H_{31}$ (mélange érythro:thréo)

EXEMPLES DE PREPARATION DE COMPOSITIONS COSMETIQUES

EXEMPLE 1C : Lotion capillaire

Cette lotion est préparée en mélangeant dans l'ordre les constituants suivants :

| | |
|---|---|
| Microdispersion à 10 % de cire obtenue à l'exemple 1 | 97,3 g |
| Parahydroxybenzoate de méthyle | 0,1 g |
| Dérivé d'imidazolidinylurée vendu sous la dénomination SUTTON CABS | 0,2 g |
| NaOH q.s.p. pH = 6,8 | |
| Eau q.s.p. | 100 g |

Cette lotion est appliquée après lavage et séchage des cheveux à raison d'environ 2 g par tête. La chevelure a du volume. Les cheveux ont du corps et sont bien disciplinés.

EXEMPLE 2C : Lotion capillaire

On opère, comme dans l'exemple 1C, avec les constituants suivants :

| | |
|---|---|
| Microdispersion à 15 % de cire obtenue à l'exemple 15<br>Para hydroxybenzoate de méthyle<br>GERMALL 115<br>NaOH q.s.p pH = 7<br>Eau q.s.p | 70 g<br>0,15g<br>0,2 g<br><br>100 g |

Cette lotion est appliquée sur des cheveux propres et séchés. Elle leur apporte du gonflant et de la brillance.

EXEMPLE 3C et 4C : Gels coiffants

Ces gels ont la composition pondérale suivante :

| | EXEMPLE N° 3C | EXEMPLE N° 4C |
|---|---|---|
| Microdispersion de l'exemple 22 | 12 g | 12 g |
| Méthyl hydroxypropyl cellulose commercialisé sos le nom de de METHOCEL 60 HG par Dow Corning | 2,20 g | - |
| Hydroxyéthyl cellulose commercialisé sous le nom NATROSOL 250 HHR par Aqualon | - | 1,20 g |
| p.hydroxybenzoate de méthyle | 0,15 g | 0,15 g |
| DOWICIL 200 - Dow Corning | 0,3 g | 0,3 g |
| Eau q.s.p. | 100 g | 100 g |

On applique ces gels coiffants sur cheveux propres et secs sur toute la longueur des cheveux y compris les racines, à raison de 2 à 5 g par tête.

Les gels s'étalent facilement dans les cheveux qui ont un toucher soyeux et du volume, du gonflant et de la discipline.

EXEMPLE 5C : Lotion capillaire

Cette lotion est préparée en mélangeant dans l'ordre les constituants suivants :

| | |
|---|---|
| Microdispersion de cire obtenue à l'exemple 41<br>Parahydroxybenzoate de méthyle<br>GERMALL 115<br>NaOH q.s.p pH = 6,8<br>Eau q.s.p | 95 g<br>0,15 g<br>0,2 g<br><br>100 g |

On applique cette solution (environ 2g/tête) raie par raie sur des cheveux secs non lavés et on laisse en contact 5 minutes avant d'effectuer un shampoing. les cheveux présentent alors du volume et de la discipline. Les mèches sont colorés en jaune.

EXEMPLE 6C : Lotion capillaire

On opère, comme dans l'exemple 1C, avec les constituants suivants :

| | |
|---|---|
| Microdispersion de cire obtenue à l'exemple 43 | 97 g |
| Para hydroxybenzoate de méthyle | 0,1 g |
| GERMALL 115 | 0,2 g |
| NaOH q.s.p pH = 6,8 | |
| Eau q.s.p | 100 g |

On applique cette composition sur cheveux propres et secs, sur les racines et sur la longueur des cheveux, à raison de 2 g par tête.

Les cheveux sont doux, ils présentent de la discipline et du gonflant. L'application de la composition confère en plus aux cheveux un effet de protection vis-à-vis du rayonnement UV.

EXEMPLE 7C

Cette lotion est préparée en mélangeant dans l'ordre les constituants suivants :

| | |
|---|---|
| Microdispersion de cire obtenue à l'exemple 46a | 95 g |
| Para hydroxybenzoate de méthyle | 0,15 g |
| GERMALL 115 | 0,2 g |
| NaOH q.s.p pH = 6,9 | |

L'application de cette composition sur cheveux secs et propres apporte à la coiffure du gonflant et de la brillance.

EXEMPLE 8C

On opère, comme dans l'exemple 1C, avec les constituants suivants :

| | |
|---|---|
| Microdispersion de cire obtenue à l'exemple 49 | 97 g |
| Para hydroxygenzoate de méthyle | 0,1 g |
| GERMALL 115 | 0,2 g |
| NaOH q.s.p pH = 7 | |
| Eau q.s.p | 100 g |

Cette composition est appliquée après un shampooing. Les cheveux présentent de la tenue, de la discipline et du corps.

**Revendications**

**1.** Composition fluide se présentant sous la forme d'une dispersion de cire dans un véhicule liquide constitué d'une seule phase, dans laquelle la phase dispersée est une microdispersion stable constituée de particules de dimensions inférieures à 0,5µm, lesdites particules sont constituées essentiellement d'un mélange d'au moins une cire, d'au moins un agent émulsionnant et éventuellement d'au moins une huile et/ou d'au moins un ingrédient actif liposoluble, ledit mélange ayant un point de fusion finissante supérieur à 50 °C et inférieur à 100 °C, ladite composition contient, en poids, de 0,1 à 40 % de cire et de 0,01 à 25 % dudit agent émulsionnant, ledit agent émulsionnant est un émulsionnant cationique, et la concentration de ladite huile, lorsqu'elle est présente, est inférieure ou égale à 30 % en poids par rapport au poids de la cire ou du mélange de cires.

**2.** Composition selon la revendication 1, caractérisée par le fait qu'elle est exempte de tensioactif non ionique.

**3.** Composition selon la revendication 1 ou 2, caractérisée par le fait qu'elle est exempte de tensioactif anionique.

**4.** Composition selon l'une quelconque des revendications précédentes, caractérisée par le fait qu'elle est exempte de tensioactifs anioniques et non ioniques.

**5.** Composition selon l'une quelconque des revendications précédentes, caractérisée par le fait que ledit agent émulsionnant est un dérivé d'ammonium quaternaire.

**6.** Composition selon l'une quelconque des revendications précédentes, caractérisée par le fait que ledit agent émulsionnant a une valeur de HLB comprise entre 11 et 16.

**7.** Composition selon l'une quelconque des revendications précédentes, caractérisée par le fait que l'agent émulsionnant cationique est présent à une concentration de 0,1 à 10 %.

**8.** Composition selon l'une quelconque des revendications précédentes, caractérisée par le fait que la concentration de l'huile, lorsqu'elle est présente, est inférieure à 10 % en poids par rapport au poids de la cire ou du mélange de cires.

**9.** Composition selon l'une quelconque des revendications précédentes, caractérisée par le fait qu'elle est exempte d'huile.

**10.** Composition selon l'une quelconque des revendications précédentes, caractérisée par le fait que ladite cire est choisie parmi la cire de Carnauba, la cire de Candelilla, la cire d'Alfa, ou leurs mélanges.

**11.** Composition selon l'une quelconque des revendications précédentes, caractérisée par le fait que la cire contient, outre de la cire de Carnauba ou de la cire de Candelilla ou leurs mélanges, une autre cire ou un mélange d'autres cires.

**12.** Composition selon la revendication 11, caractérisée par le fait que ladite autre cire est une cire de paraffine, l'ozokérite, la cire de jojoba hydrogénée, la cire de riz ou la cire d'abeille éventuellement estérifiée.

**13.** Composition selon la revendication 12, caractérisée par le fait que ladite autre cire est un céramide.

**14.** Composition selon l'une quelconque des revendications 11 à 13 caractérisée par le fait que la proportion pondérale de cire de Carnauba et/ou de Candelilla est supérieure ou égale à 50 % par rapport au poids total de cire.

**15.** Composition selon l'une quelconque des revendications précédentes, caractérisée par le fait que ledit véhicule liquide contient de 70 à 100 % d'eau, par rapport au poids de la phase liquide.

**16.** Composition selon l'une quelconque des revendications précédentes, caractérisée par le fait que ledit véhicule aqueux est constitué par de l'eau.

**17.** Composition selon l'une quelconque des revendications précédentes, caractérisée par le fait que le rapport pondéral cire/émulsionnant est dans la gamme de 1 à 20.

**18.** Composition selon la revendication 17, caractérisée par le fait que ledit rapport est dans la gamme de 2 à 10.

**19.** Composition selon l'une quelconque des revendications précédentes, caractérisée par le fait qu'au moins un composé amphiphile non émulsionnant des cires, tel que le cholestérol et les alcools gras ayant au moins 12 atomes de carbone est associé à la cire, à une concentration pouvant aller jusqu'à 30 %, et en particulier jusqu'à 10 % en poids par rapport à la cire.

**20.** Composition selon l'une quelconque des revendications précédentes, caractérisée par le fait que la concentration des ingrédients actifs liposolubles, lorsqu'ils sont présents, peut aller jusqu'à 30 % et en

particulier jusqu'à 10 %, en poids par rapport à la cire ou au mélange de cires.

**21.** Composition selon l'une quelconque des revendications 1 à 18, caractérisée par le fait que la proportion pondérale de cire, dans les particules (agents émulsionnants mis à part), est supérieure à 90 %, et en particulier supérieure à 95 %.

**22.** Composition selon la revendication 19, caractérisée par le fait que la proportion pondérale de cire et de composés amphiphiles non émulsionnants dans les particules (agents émulsionnants mis à part), est supérieure à 90 %, et en particulier supérieure à 95%.

**23.** Composition selon l'une quelconque des revendications précédentes, caractérisée par le fait que lesdits ingrédients actifs liposolubles sont choisis parmi les colorants liposolubles et les substances liposolubles capables de conférer une protection contre les effets nocifs du rayonnement ultraviolet.

**24.** Procédé de préparation d'une composition telle que définie dans l'une quelconque des revendications précédentes, caractérisé par le fait que l'on chauffe le mélange de cire et d'émulsionnant, additionné éventuellement d'huile et/ou des substances liposolubles, à une température supérieure à la température de fusion du mélange et non supérieure à 100 °C, éventuellement en présence d'une partie de l'eau, jusqu'à fusion complète, que l'on ajoute progressivement l'eau, ou le restant de l'eau, portée à une température au moins égale à ladite température, en agitant, jusqu'à formation d'une microémulsion de cire dans une phase continue aqueuse, qui par refroidissement jusqu'à la température ambiante conduit à une microdispersion de cire.

**25.** Utilisation d'une composition telle que définie dans l'une quelconque des revendications 1 à 23, comme agent d'entretien auto-lustrant pour articles en cuir, revêtements de sol ou meubles, ou comme composition cosmétique ou support de composition cosmétique pour cheveux.

**26.** Procédé de traitement cosmétique des cheveux, destiné notamment à améliorer la tenue et le volume de la coiffure et/ou à supprimer ou retarder l'apparition de l'aspect gras des cheveux, caractérisé par le fait que l'on applique, au moins sur la partie des cheveux voisine de la racine, une composition telle que définie dans l'une quelconque des revendications 1 à 23, en quantité suffisante pour imprégner les cheveux ou parties de cheveux à traiter.

## Claims

**1.** Fluid composition which is provided in the form of a dispersion of wax in a liquid vehicle consisting of a single phase, in which the dispersed phase is a stable microdispersion consisting of particles less than 0.5 µm in size, the said particles consisting essentially of a mixture of at least one wax, of at least one emulsifying agent and optionally of at least one oil and/or of at least one lipid-soluble active ingredient, the said mixture having a final melting point above 50 °C and below 100 °C, the said composition contains, by weight, from 0.1 to 40% of wax and from 0.01 to 25% of the said emulsifying agent, the said emulsifying agent is a cationic emulsifying agent, and the concentration of the said oil, when it is present, is less than or equal to 30% by weight relative to the weight of the wax or of the mixture of waxes.

**2.** Composition according to Claim 1, characterized in that it is free of nonionic surfactant.

**3.** Composition according to Claim 1 or 2, characterized in that it is free of anionic surfactant.

**4.** Composition according to any one of the preceding claims, characterized in that it is free of anionic and nonionic surfactants.

**5.** Composition according to any one of the preceding claims, characterized in that the said emulsifying agent is a quaternary ammonium derivative.

**6.** Composition according to any one of the preceding claims, characterized in that the said emulsifying agent has an HLB value between 11 and 16.

**7.** Composition according to any one of the preceding claims, characterized in that the cationic emulsifying agent is present at a concentration of from 0.1 to 10%.

**8.** Composition according to any one of the preceding claims, characterized in that the concentration of the oil, when it is present, is less than 10% by weight relative to the weight at the wax or of the mixture of waxes.

**9.** Composition according to any one of the preceding claims, characterized in that it is free of oil.

**10.** Composition according to any one of the preceding claims, characterized in that the said wax is chosen from carnauba wax, candelilla wax, esparto wax or mixtures thereof.

**11.** Composition according to any one of the preceding claims, characterized in that the wax contains, besides carnauba wax or candelilla wax or mixtures thereof, another wax or a mixture of other waxes.

**12.** Composition according to Claim 11, characterized in that the said other wax is a paraffin wax, ozokerite, hydrogenated jojoba wax, rice wax or beeswax, which may optionally be esterified.

**13.** Composition according to Claim 12, characterized in that the said other wax is a ceramide.

**14.** Composition according to any one of Claims 11 to 13, characterized in that the weight proportion of carnauba wax and/or of candelilla wax is greater than or equal to 50% relative to the total weight of wax.

**15.** Composition according to any one of the preceding claims, characterized in that the said liquid vehicle contains from 70 to 100% of water relative to the weight of the liquid phase.

**16.** Composition according to any one of the preceding claims, characterized in that the said aqueous vehicle consists of water.

**17.** Composition according to any one of the preceding claims, characterized in that the wax/emulsifying agent weight ratio is in the range from 1 to 20.

**18.** Composition according to Claim 17, characterized in that the said ratio is in the range from 2 to 10.

**19.** Composition according to any one of the preceding claims, characterized in that at least one amphiphilic compound which dose not emulsify the waxes, such as cholesterol and fatty alcohols having at least 12 carbon atoms, is combined with the wax, in a concentration which may range up to 30%, and in particular up to 10%, by weight relative to the wax.

**20.** Composition according to any one of the preceding claims, characterized in that the concentration of the lipid-soluble active ingredients, when they are present, may range up to 30%, and in particular up to 10%, by weight relative to the wax or to the mixture of waxes.

**21.** Composition according to any one of Claims 1 to 18, characterized in that the weight proportion of wax in the particles (except for the emulsifying agents) is greater than 90% and in particular greater than 95%.

**22.** Composition according to Claim 19, characterized in that the weight proportion of wax and of non-emulsifying amphiphilic compounds in the particles (except for the emulsifying agents) is greater than 90% and in particular greater than 95%.

**23.** Composition according to any one of the preceding claims, characterized in that the said lipid-soluble active ingredients are chosen from lipid-soluble dyes and lipid-soluble substances capable of conferring protection against the harmful effects of ultraviolet radiation.

**24.** Process for the preparation of a composition as defined in any one of the preceding claims, characterized in that the mixture of wax and emulsifying agent, to which is optionally added oil and/or

lipid-soluble substances, is heated to a temperature above the melting point of the mixture and not above 100 °C, optionally in the presence of some of the water, until fusion is complete, and in that water, or the remainder of the water, which has been brought to a temperature at least equal to the said temperature, is progressively added with stirring until a microemulsion of wax in a continuous aqueous phase is formed, which microemulsion, on cooling to room temperature, gives a microdispersion of wax.

**25.** Use of a composition as defined in any one of Claims 1 to 23 as a self-lustreing maintenance agent for leather articles, floor coverings or furniture coverings, or as a cosmetic composition or cosmetic composition support for hair.

**26.** Cosmetic hair treatment process which is intended in particular to enhance the hold and the volume of the hairstyle and/or to eliminate or to delay the appearences of the greasy aspect of the hair, characterized in that a composition as defined in any one of Claims 1 to 23 is applied to at least the part of the hair near to the roots, in a sufficient amount to impregnate the hair or parts of the hair to be treated.

**Patentansprüche**

**1.** Flüssige Zubereitung, die in Form einer Wachsdispersion in einem flüssigen Träger vorliegt, welcher aus einer einzigen Phase besteht, worin die dispergierte Phase eine stabile Mikrodispersion darstellt, welche aus Teilchen mit Abmessungen von weniger als 0,5 µm gebildet ist, wobei diese Teilchen im wesentlichen aus einem Gemisch mindestens eines Wachses, mindestens eines Emulgiermittels und gegebenenfalls mindestens eines Öles und/oder mindestens eines fettlöslichen Wirkstoffes besteht, diese Mischung einen Endschmelzpunkt oberhalb von 50 °C und unterhalb von 100 °C besitzt und die Zubereitung, in Form des Gewichtes ausgedrückt, zwischen 0,1 und 40% Wachs sowie zwischen 0,01 und 25% Emulgiermittel enthält, wobei das Emulgiermittel einen kationischen Emulgator darstellt und die Konzentration des Öls, im Falle seiner Anwesenheit weniger oder gleich 30 Gewichtsprozent in Bezug auf das Gewicht des Wachses oder des Wachsgemisches beträgt.

**2.** Zubereitung gemäß Anspruch 1, dadurch gekennzeichnet, daß sie frei ist von einem nichtionischen Tensid.

**3.** Zubereitung gemäß Anspruch 1 oder 2, dadurch gekennzeichnet, daß sie frei ist von einem anionischen Tensid.

**4.** Zubereitung gemäß einem der vorstehenden Ansprüche, dadurch gekennzeichnet, daß sie frei ist von anionischen und nichtionischen Tensiden.

**5.** Zubereitung gemäß einem der vorstehenden Ansprüche, dadurch gekennzeichnet, daß der Emulgator ein quaternäres Ammoniumderivat darstellt.

**6.** Zubereitung gemäß einem der vorstehenden Ansprüche, dadurch gekennzeichnet, daß der Emulgator einen HLB-Wert zwischen 11 und 16 aufweist.

**7.** Zubereitung gemäß einem der vorstehenden Ansprüche, dadurch gekennzeichnet, daß der kationische Emulgator in einer Konzentration zwischen 0,1 und 10% vorliegt.

**8.** Zubereitung gemäß einem der vorstehenden Ansprüche, dadurch gekennzeichnet, daß die Konzentration an Öl im Falle seiner Anwesenheit weniger als 10 Gewichtsprozent in Bezug auf das Gewicht des Wachses oder des Wachsgemisches beträgt.

**9.** Zubereitung gemäß einem der vorstehenden Ansprüche, dadurch gekennzeichnet, daß sie frei ist von Öl.

**10.** Zubereitung gemäß einem der vorstehenden Ansprüche, dadurch gekennzeichnet, daß das Wachs aus Carnaubawachs, Candelillawachs, Alfawachs oder deren Gemischen ausgewählt ist.

**11.** Zubereitung gemäß einem der vorstehenden Ansprüche, dadurch gekennzeichnet, daß das Wachs außer Carnaubawachs oder Candelillawachs oder deren Gemischen noch ein weiteres Wachs oder eine Mischung anderer Wachse enthält.

**12.** Zubereitung gemäß Anspruch 11, dadurch gekennzeichnet, daß das zusätzliche andere Wachs ein Paraffinwachs, Ozokerit, hydriertes Jojobawachs, Reiswachs oder Bienenwachs in gegebenenfalls veresterter Form darstellt.

**13.** Zubereitung gemäß Anspruch 12, dadurch gekennzeichnet, daß das zusätzliche weitere Wachs ein Ceramid darstellt.

**14.** Zubereitung gemäß einem der Ansprüche 11-13, dadurch gekennzeichnet, daß das Gewichtsverhältnis des Carnaubawachses und/oder Candelillawachses mehr als oder gleich 50% in Bezug auf das Gesamtgewicht des Wachses beträgt.

**15.** Zubereitung gemäß einem der vorstehenden Ansprüche, dadurch gekennzeichnet, daß der flüssige Träger zwischen 70 und 100% Wasser in Bezug auf das Gewicht der Flüssigphase enthält.

**16.** Zubereitung gemäß einem der vorstehenden Ansprüche, dadurch gekennzeichnet, daß der wässrige Träger aus Wasser besteht.

**17.** Zubereitung gemäß einem der vorstehenden Ansprüche, dadurch gekennzeichnet, daß das Gewichtsverhältnis Wachs zu Emulgator im Bereich zwischen 1 und 20 liegt.

**18.** Zubereitung gemäß Anspruch 17, dadurch gekennzeichnet, daß das Gewichtsverhältnis im Bereich zwischen 2 und 10 liegt.

**19.** Zubereitung gemäß einem der vorstehenden Ansprüche, dadurch gekennzeichnet, daß mindestens eine amphiphile, die Wachse nicht emulgierende Verbindung wie z.B. Cholesterin oder Fettalkohole mit mindestens 12 Kohlenstoffatomen mit dem Wachs assoziiert sind, und zwar in einer Konzentration, welche bis zu 30%, insbesondere bis zu 10 Gewichtsprozent in Bezug auf das Wachs betragen kann.

**20.** Zubereitung gemäß einem der vorstehenden Ansprüche, dadurch gekennzeichnet, daß die Konzentration an fettlöslichen Wirkstoffen im Falle ihrer Anwesenheit bis zu 30 Gewichtsprozent und insbesondere bis zu 10 Gewichtsprozent in Bezug auf das Wachs oder Wachsgemisch betragen kann.

**21.** Zubereitung gemäß einem der Ansprüche 1-18, dadurch gekennzeichnet, daß das Gewichtsverhältnis des Wachses in den Teilchen ( abgesehen von den Emulgatoren) mehr als 90%, und insbesondere mehr als 95% beträgt.

**22.** Zubereitung gemäß Anspruch 19, dadurch gekennzeichnet, daß der Gewichtsanteil an Wachs und der nichtemulgierenden amphiphilen Verbindungen in den Teilchen ( abgesehen von den Emulgatoren) mehr als 90%, und insbesondere mehr als 95% beträgt.

**23.** Zubereitung gemäß einem der vorstehenden Ansprüche, dadurch gekennzeichnet, daß die fettlöslichen Wirkstoffe unter fettlöslichen Farbstoffen und solchen fettlöslichen Substanzen ausgewählt sind, welche zu einer Schutzwirkung vor den schädlichen Auswirkungen der UV-Strahlung befähigt sind.

**24.** Verfahren zur Herstellung einer Zubereitung gemäß Definition nach einem der vorstehenden Ansprüche, dadurch gekennzeichnet, daß man ein Gemisch aus Wachs und Emulgiermittel, gegebenenfalls unter Zusatz von Öl und/oder fettlöslichen Substanzen bei einer Temperatur erhitzt, welche oberhalb der Schmelztemperatur des Gemisches liegt, jedoch nicht mehr als 100 °C beträgt, gegebenenfalls in Anwesenheit einer Teilmenge an Wasser bis zum vollständigen Schmelzen allmählich Wasser hinzufügt oder die Restmenge an Wasser, welche man einer Temperatur oder mindestens unter Rühren bei der Temperatur gehalten wird, hinzugibt, bis sich eine Wachsmikroemulsion in einer kontinuierlich wässrigen Phase bildet, die unter Abkühlung bis herab auf Raumtemperatur bis zur Mikrodispersion des Wachses führt.

**25.** Verwendung einer Zubereitung gemäß Definition nach einem der Ansprüche 1-23 als selbstglanzbildendes Pflegemittel für Lederartikel, Bodenbeläge oder Möbelverkleidungen oder als kosmetische Zubereitung oder als Trägermaterial in kosmetischen Haarpflegezubereitungen.

**26.** Verfahren zur kosmetischen Haarbehandlung, welches insbesondere zur Verbesserung des Halts und Volumens der Frisur und/oder zur Beseitigung oder zum verzögerten Auftreten des fettigen Aussehens der Haare bestimmt ist, dadurch gekennzeichnet, daß mindestens auf einem Teil des der Wurzel nahestehenden Haargrundes eine Zubereitung gemäß Definition nach einem der Ansprüche 1-23 in einer Menge zur Anwendung gebracht wird, welche ausreicht, die zu behandelnden Haare oder Haarpartien zu benetzen.